# EUROPEAN PATENT SPECIFICATION

(11) **EP 0 491 173 B1**
(45) Date of publication and mention of the grant of the patent: **14.02.1996**
(21) Application number: 91119656.6
(22) Date of filing: 18.11.1991
(51) Int. Cl.: C07K 5/06

(54) **Method of producing hydrochloride of alpha-L-aspartyl-L-phenylalanine methyl ester**
Verfahren zur Herstellung von Alpha-L-aspartyl-L-phenylalanyl-methylester
Méthode pour le production du chlorhydrate de alpha-L-aspartyl-L-phenylalanine methylester

(30) Priority: 20.11.1990 JP 315568/90
(43) Date of publication of application: 24.06.1992
(73) Proprietor: Ajinomoto Co., Inc., Tokyo 104 (JP)
(72) Inventor: Kato, Toshihisa, c/o Central Research Laboratories, Kawasaki-shi, Kanagawa-ken (JP); Takeda, Hideo, c/o Central Research Laboratories, Kawasaki-shi, Kanagawa-ken (JP); Kano, Mikiya, c/o Central Research Laboratories, Kawasaki-shi, Kanagawa-ken (JP); Takemoto, Tadashi, c/o Central Research Lab., Kawasaki-shi, Kanagawa-ken (JP)
(74) Representative: Strehl Schübel-Hopf Groening & Partner

(56) References cited:
- EP-A- 0 200 311
- EP-A- 0 227 301

## Description

### Field of the Invention

This invention relates to a method of producing hydrochloride of α-L-aspartyl-L-phenylalanine methyl ester ( hereinafter abbreviated as "α-APM" in some cases ).

### Prior Art

α-APM is known as an excellent sweetening agent, and is generally produced by the method which comprises condensing an N-protected-L-aspartic acid anhydride with L-phenylalanine methyl ester ( hereinafter abbreviated as "PM" ) to form an N-protected-L-aspartyl-L-phenylalanine methyl ester, and eliminating the N-protective group from this reaction product, thereby giving the objective α-APM ( refer to U.S. Patent No.3,786,039 ).

As the N-protective group, is generally used formyl group ( hereinafter abbreviated as "F" or "For" in some cases ) in industrial production. The known methods to eliminate an N-formyl group are the method using dilute hydrochloric acid, the method using hydrogen peroxide [ G. Losse, et al.; Ann. Chem., 636, 140 ( 1960 )], the method using hydrazine ( P. Lefranicier, et al.; Bull. Soc. Chim. France, 1965, 368 ), and the method using hydroxylamine [ R. Geiger; Chem. Ber., 101, 3386 ( 1968 )].

### Problems to be Solved by the Invention

Of the methods described above, those using hydrochloric acid are excellent as an industrial process because of the simple operations involved and the low cost.

For example, acidic hydrolysis by heating with dilute hydrochloric acid is applied to N-formyl amino acids [ V. du Vigneaud, et al.; J. Biol. Chem., 98, 577 ( 1932 )], and a method of holding in a methanolic solution of hydrogen chloride of 0.5N or lower concentration for 48 hours is applied to N-formyl peptides [ J.C. Sheehan, et al.; J. Am. Chem. Soc., 80, 1158 ( 1958 )] in order to avoid hydrolysis of the peptide linkage. However, when treating a peptide containing ester and carboxyl groups, in addition to the amino group protected with a formyl group, the acidic hydrolysis method has the defect of hydrolyzing the ester group and the peptide linkage, and the acidic alcoholysis method has the defect of esterifying the free carboxyl group.

Under the circumstances, the present inventors formerly invented a new method free of the above defects, in which the formyl group is eliminated by contact with a mixture of methanol and high-concentration hydrochloric acid at 0 to 40°C, and the α-L-aspartyl-L-phenylalanine methyl ester thus produced is separated out in the form of sparingly soluble hydrochloride ( U.S. Patent No.4,684,745 ).

This method, however, involves the problem that, unless the mixture of N-formyl-α-L-aspartyl-L-phenylalanine methyl ester, methanol and high-concentration hydrochloric acid is held at a high temperature for a short time, the hydrochloride separated out contains the unreacted N-formyl compound in an amount of several percentages ( 1 to 2% in many cases ). Hence, a number of purification steps are required to obtain the free ester ( aspartame ) containing no N-formyl compound from the separated hydrochloride.

Accordingly, it is necessary to hold the mixture at a high temperature for a short time in order to industrially produce high-quality hydrochloride of α-L-aspartyl-L-phenylalanine methyl ester containing no N-formyl-α-L-aspartyl-L-phenylalanine methyl ester. This requires special equipment and complicated operations.

EP-A-0 200 311 relates to a process for preparing α-L-aspartyl-L-phenylalanine methyl ester or its hydrochloride, wherein N-formyl-L-aspartic acid anhydride and L-phenylalanine are condensed in water or in water containing methanol to form N-formyl-α-L-aspartyl-L-phenylalanine. The reaction mixture is then acidified with hydrochloric acid in the presence of methanol whereby α-L-aspartyl-L-phenylalanine methyl ester hydrochloride is deposited which may then be separated and neutralised.

EP-A-0 227 301 relates to a process for the preparation of N-formyl-α-L-aspartyl-L-phenylalanine methyl ester by reacting N-formyl-L-aspartic acid anhydride with L-phenylalanine methyl ester in a reaction solvent in the presence of acetic acid and formic acid in a complete mixing type reaction process.

Thus, the object of this invention is to establish an industrial method for producing hydrochloride of α-L-aspartyl-L-phenylalanine methyl ester containing practically no N-formyl-α-L-aspartyl-L-phenylalanine methyl ester without having to hold the mixture at a high temperature for a short time in the deformylation step.

### Means to Solve the Problems

Intensive studies to overcome these problems have led us to find that, if a mixture of N-formyl-α-L-aspartyl-L-phenylalanine methyl ester, water and methanol is treated in the presence of hydrochloric acid by a continuous reaction process of complete mixing type or by a process involving both continuous and batch reaction steps of complete mixing type, wherein said continuous reaction process is performed at a temperature in the range of 15°C to 40 °C, preferably 20°C to 35°C, hydrochloride of α-L-aspartyl-L-phenylalanine methyl ester containing no N-formyl-α-L-aspartyl-L-phenylalanine methyl ester can be surprisingly obtained without having to hold the mixture at a high temperature for a short time. This invention was accomplished on the basis of this finding.

To be more specific, in the method of U.S. Patent No.4,684,745 ( eliminating the formyl group by contact with methanol and high-concentration hydrochloric acid at a temperature of 0 to 40°C, and separating out α-L-aspartyl-L-phenylalanine methyl ester thus produced in the form of sparingly soluble hydrochloride ), the objective α-APM hydrochloride is separated out in the presence of a large quantity of starting material, For-α-APM, and it is therefore unavoidable that For-α-APM is also separated out together with hydrochloride of α-APM in an amount of about several percentages.

Other types of α-AP derivatives, such as α-L-aspartyl-L-phenylalanine ( hereinafter abbreviated as "α-AP" in some cases ), α-L-aspartyl-L-phenylalanine dimethyl ester ( hereinafter abbreviated as "α-APM₂" in some cases ) and α-L-aspartyl-L-phenylalanine aspartic acid β-methyl ester ( hereinafter abbreviated as "α-AP(M)" in some cases ), can be easily removed, even when included in hydrochloride of α-APM in an amount of several percentages, in the step of conversion to free ester. However, when For-α-APM is included in an amount of 1 to 2%, a number of purification steps is required to obtain free ester not containing the same ( aspartame ).

As a result of intensive studies, the present inventors discovered that treatment of a mixture of For-α-APM, methanol and high-concentration hydrochloric acid by a continuous reaction process of complete mixing type or by a process involving both continuous and batch reaction steps gives hydrochloride of α-APM containing no For-α-APM, thus accomplishing this invention.

There is no specific limitation upon the concentration of F-α-APM to be fed, but a concentration in the range from 0.3 to 1.8 M/l is preferable. The amount of methanol should be 5 to 60 vol.%, preferably 10 to 30 vol.%, based on the volume of hydrochloric acid. Methanol may be fed to a reactor as a mixture with a solution or slurry of F-α-APM, or these may be fed separately. Hydrochloric acid should be added in such an amount that its concentration in the deformylation reaction system will be in the range of from 2 to 8N, preferably from 2.5 to 4.5N, and should be added to the reactor separately from F-α-APM.

The reactor should be held at a temperature of 15 to 40°C, preferably from 20 to 35°C, and the volume of high-concentration hydrochloric acid ( or a slurry of α-APM hydrochloride in a mixture of hydrochloric acid and methanol ) to be previously put in the reactor should be at least 50 times, preferably at least 100 times, as much as the volume of F-α-APM solution to be fed or the volume of its slurry added per hour. The degree of agitation depends on the size of the crystallizer and reactor, the type of agitator and so on. However, agitation is performed in such a way that results in a strong mixing state in the vessel. The total reaction time is about one to six days; and the reaction is actually carried out by a cascade process (a continous process performing reaction and crystallization in several continuous steps by the use of several vessels), or by a process involving both continuous and batch reaction steps of complete mixing type.

The following Examples will further illustrate the invention.

### Example 1

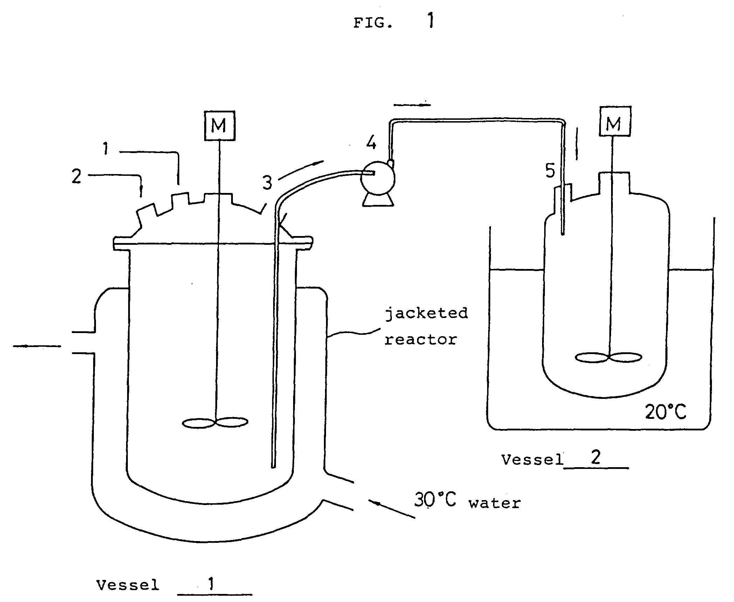

In a jacketed reactor with an internal volume of three liters as shown in Fig. 1 ( Vessel 1 ), was put one liter of 3.5N-HCl, and 100 g of α-APM·HCl was then added, producing a preliminary slurry containing seed crystals which ensure stable crystallization of α-APM·HCl formed by deformylation of F-α-APM added in the initial step.

This reactor was maintained at a temperature of 25°C, and a slurry formed by mixing 118 g F-α-APM, 326 g water and 46 ml methanol and held at room temperature was then continuously fed to the reactor at a rate of 60 g/h. Separately, 35%-HCl was fed to the same reactor at a rate of 23.5 g/h to effect the reaction of complete mixing type under stirring, and the slurry formed in the reactor was withdrawn into Vessel 2 by operating the slurry pump so that the volume of the slurry left in the reactor would be always three liters. Since the slurry feeding was completed in eight hours, this operation was repeated three times in 24 hours and was continued for six days. The residence time in Vessel 1 was aproximately 42 hours.

The temperature of Vessel 2 was maintained at 20°C. After the content in Vessel 1 reached a volume of three liters, the slurry was withdrawn at a rate of 83.5 g/h ( total amount withdrawn in 24 hours: 2000 g, about 1700 ml ), the transferred slurry was subjected to batch crystallization once every day ( stirring at 20°C for two days, followed by stirring at 5°C for three hours ), and the crystals thus separated out were collected. While the reaction in Vessel 1 was of a continuous type, the present Vessel 2 was replaced by a new empty vessel 2 once a day. Analytical values of the crystals obtained on the third day from the start of withdrawal and after that (at the time in which the system is considered to be stabilized) are shown in the table below. Analysis was performed by the use of an amino acid analyzer and by high performance liquid chromatography.

### Analytical Values in Example 1

Analytical values of α-AP derivatives in wet crystals obtained by batch crystallization in Vessel 2

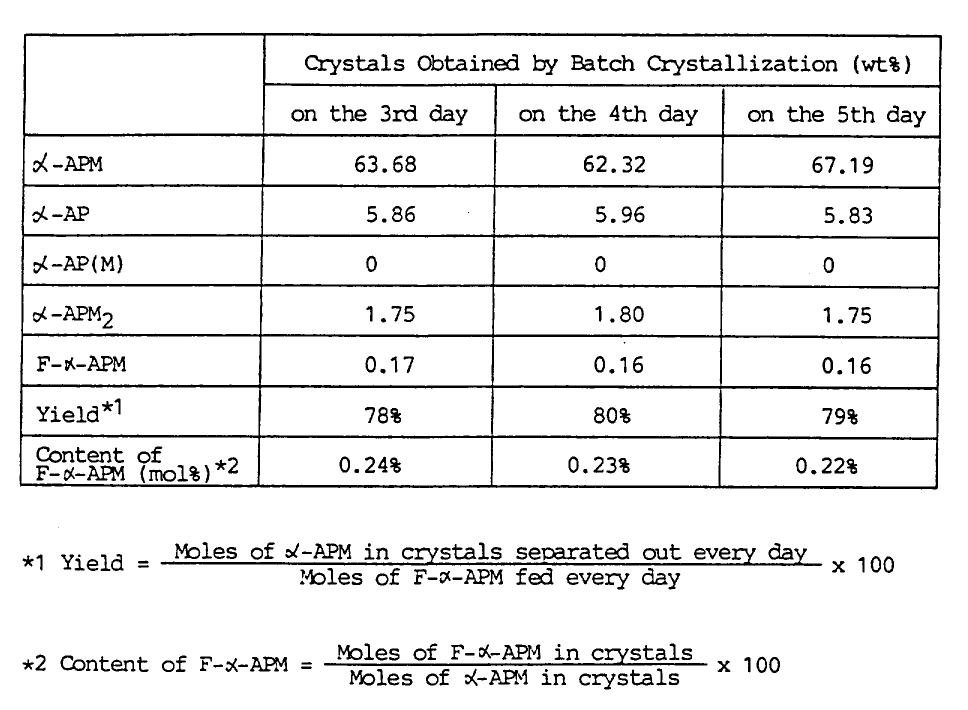

### Example 2

In a reactor whith an internal volume of three liters as shown in Fig. 1, was put a preliminary slurry in the same way as in Example 1, and the reactor was maintained at a temperature of 30°C.

The reaction was then carried out in quite the same way as in Example 1, the slurry withdrawn into Vessel 2 ( held at 20°C ) after three, four and five days was subjected to batch crystallization ( stirring at 20°C for two days and then at 5°C for three hours ), and the crystals thus separated out were collected. Analytical values of these cystals are shown in the table below.

### Analytical Values in Example 2

Analytical values of α-AP derivatives in wet crystals obtained by batch crystallization in Vessel 2

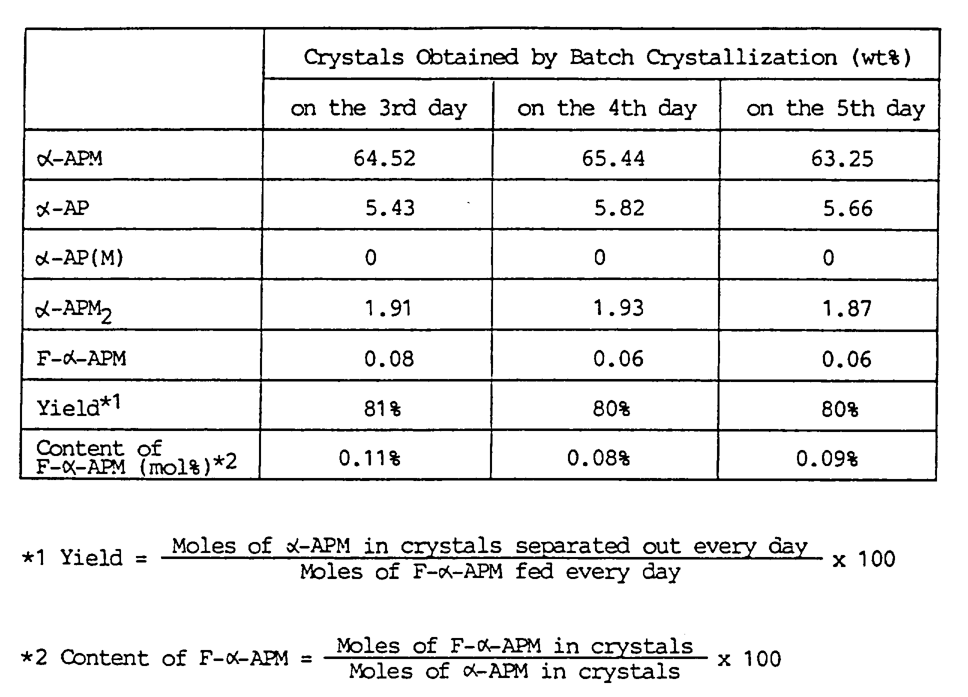

### Example 3

In a reactor with an internal volume of three liters as shown in Fig. 1, was put a preliminary slurry in the same way as in Example 1, and the reactor was maintained at a temperature of 30°C.

To this vessel, was continuously fed a mixture of 40 ml methanol and 264 g of the aqueous solution obtained in the synthesis of F-α-APM having the composition as shown below at a rate of 37 g/h while maintaining the reaction system at 40°C to avoid separation of formed crystals.

| | wt% |
|---|---|
| F-α-APM | 39.0 |
| F-β-APM | 7.0 |
| α-APM | 1.0 |

Separately, 35%-HCl was fed to the same reactor at a rate of 23.8 g/h to effect the reaction of complete mixing type under stirring. After the volume of the slurry in the reactor reached three liters, it was withdrawn into Vessel 2 at a rate of 60.8 g/h by operating the slurry pump, thereby maintaining the volume of the slurry left in the reactor at three liters. Since the slurry feeding was completed in eight hours, this operation was repeated three times in 24 hours and was continued for six days. The residence time in Vessel 1 was approximately 54 hours. The temperature of Vessel 2 was maintained at 20°C, and the transferred slurry was subjected to batch crystallization at an interval of 24 hours after the start of withdrawal ( total amount withdrawn in 24 hours: 2000 g, about 1700 ml ) the withdrawn slurry was stirred at 20°C for two days and then at 5°C for three hours and the crystals thus separated out were collected. While the reaction in Vessel 1 was of a continuous type, the present Vessel 2 was replaced by a new empty Vessel 2 once a day. Analytical values of the crystals obtained on the third day from the start of withdrawal and after that (at the time in which the system is considered to be stabilized) are shown in the table below.

### Analytical Values in Example 3

Analytical values of α-AP derivatives in wet crystals obtained by batch crystallization in Vessel 2

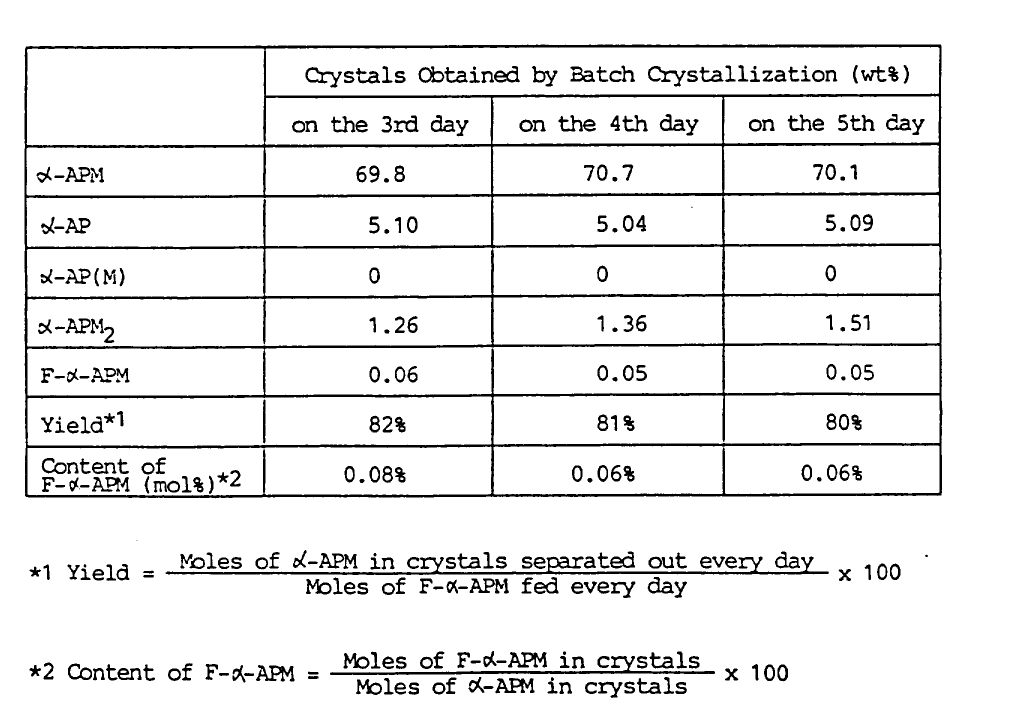

### Example 4

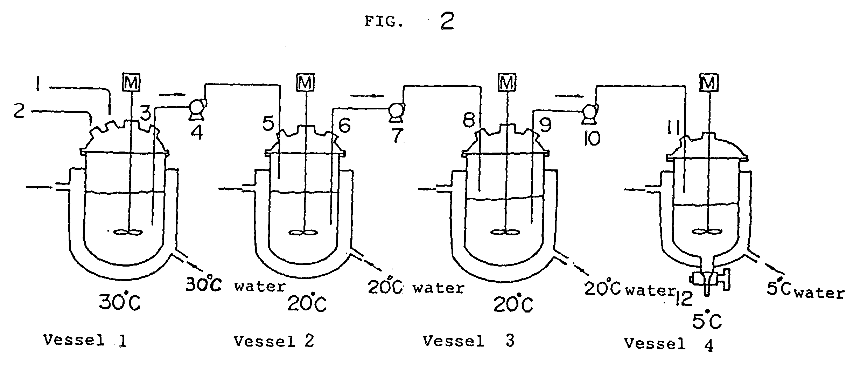

Four jacketed reactors each having an internal volume of three liters were assembled as shown in Fig. 2.

In Vessel 1, was put a preliminary slurry in the same way as in Example 1, which was maintained at a temperature of 30°C. To this vessel, was continuously fed a mixture of 40 ml methanol and 264 g of an aqueous solution obtained in the synthesis of F-α-APM having the same composition as used in Example 3 at a rate of 37 g/h three time per day, while maintaining the reaction system at 40°C to avoid separation of crystals. Separately, 35%-HCl was fed to the same reactor at a rate of 23.8 g/h to effect the reaction of complete mixing type under stirring. After the volume of the slurry in the reactor reached three liters, it was withdrawn into Vessel 2 at a rate of 60.8 g/h by operating the slurry pump, thereby maintaining the volume of slurry left in the reactor at three liters. Similarly, after the volume of the slurry in Vessel 2 reached three liters, it was withdrawn into Vessel 3 at a rate of 60.8 g/h by operating the slurry pump, thereby maintaining the volume of slurry left in Vessel 2 at three liters. After the volume of the slurry in Vessel 3 reached three liters, it was withdrawn into Vessel 4 so that the volume of the slurry left in Vessel 3 would be maintained at three liters. The slurry in Vessel 4 was held at 5°C, crystals separated out were collected every day, and the yield and composition of the same were analyzed.

The residence time of the slurry was 54.2 hours in each of Vessels 1 through 3, and 12 hours in Vessel 4. This continuous operation was continued for ten days, and the crystals obtained after eight days and thereafter (at the time in which the system is considered to be stabilized) were collected and analyzed. The results obtained are summarized in the table below.

### Analytical Values in Example 4

Analytical values of α-AP derivatives in wet crystals obtained by batch crystallization in Vessel 2

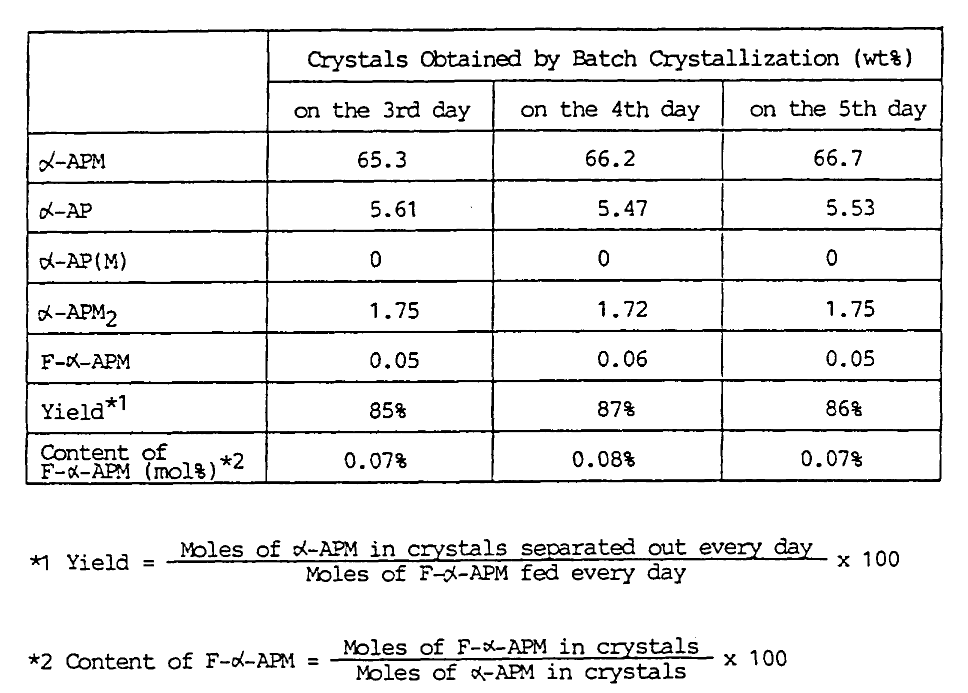

### Comparative Example 1

To a solvent mixture composed of 15 ml methanol, 25 ml water and 42 ml of 35%-HCl, was added 48.5 g For-α-APM, the resulting mixture was stirred at 25°C for four days and then at 5°C for three hours, and the crystals which separated out were collected and analyzed. The result is shown below.

| | ( wt% in wet crystals ) |
|---|---|
| α-APM | 62.16% |
| α-AP | 6.23% |
| α-AP(M) | 0 % |
| α-APM₂ | 2.01% |
| F-α-APM | 1.30% |
| Yield | 81.0 % |
| Content of F-α-APM | 1.9 mol% (vs α-APM) |

### Comparative Example 2

One hundred grams of an aqueous solution for the synthesis of F-α-APM having the composition as shown below was held at 25°C, 15 ml methanol and 64 ml of 35%-HCl were added thereto, and the mixture was stirred at 25°C for four days and then at 5°C for three hours.

| | wt% |
|---|---|
| F-α-APM | 39.0 |
| F-β-APM | 7.0 |
| α-APM | 1.0 |

The crystals which separated out were collected and analyzed. The result was as shown below.

| | ( wt% in wet crystals ) |
|---|---|
| α-APM | 62.8 % |
| α-AP | 7.35% |
| α-AP(M) | 0 % |
| α-APM₂ | 2.76% |
| F-α-APM | 1.53% |
| Yield | 74.0 % |
| Content of F-α-APM | 2.2 mol% (vs α-APM) |

### Comparative Example 3

To a solvent mixture composed of 19 ml methanol, 76 ml water and 66 ml concentrated hydrochloric acid, was added 48.3 g of F-α-APM, the resulting mixture was stirred at 25°C for four days and then at 5°C for three hours, and the crystals which separated out were collected and analyzed. The result was as shown below.

| | ( wt% in wet crystals ) |
|---|---|
| α-APM | 63.43% |
| α-AP | 7.01% |
| α-AP(M) | 0 % |
| α-APM₂ | 2.57% |
| F-α-APM | 1.50% |
| Yield | 76.0% |
| Content of F-α-APM | 2.2 mol% (vs α-APM) |

### BRIEF EXPLANATION OF DRAWINGS

Figures 1 and 2 each illustrate an example of reactor used in practicing the method of this invention.

In these figures the reference numbers have the following meaning:

### Figure 1

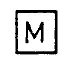 Motor
1 For-α-APM+ water + methanol feeder
2 35% Hcℓ feeder
3 Vessel 1 slurry withdrawal
4 laboratory slurry pump (e.x. FMI laboratory pump)
5 Vessel 2 feeder

### Figure 2

- M: Motor
- 1: For-α-APM reaction solution + methanol mixture feeder
- 2: 3 5 % -Hcl feeder
- 3: Vessel 1 slurry withdrawal
- 4,7,10: laboratory slurry pump
- 5: Vessel 2 feeder
- 6: Vessel 2 slurry withdrawal
- 8: Vessel 3 feeder
- 9: Vessel 3 slurry withdrawal
- 11: Vessel 4 feeder
- 12: Vessel 4 slurry withdrawal

## Claims

1. A method of producing the hydrochloride of α-L-aspartyl-L-phenylalanine methyl ester, which comprises eliminating the formyl group from N-formyl-α-L-aspartyl-L-phenylalanine methyl ester in the presence of methanol and high concentration hydrochloric acid by a continuous reaction process of complete mixing type or by a process involving both continuous and batch reaction steps of complete mixing type, and separating α-L-as-partyl-L-phenylalanine methyl ester thus produced in the form of the sparingly soluble hydrochloride, wherein said continuous reaction process is performed at a temperature in the range of 15°C to 40 °C.

2. The method according to Claim 1, wherein said continuous reaction process is performed at a temperature in the range of 20°C to 35°C.

## Patentansprüche

1. Verfahren zur Herstellung des Hydrochlorids von α-L-Aspartyl-L-phenylalanin-methylester, das darin besteht, daß die Formylgruppe aus N-Formyl-α-L-aspartyl-L-phenylalaninmethylester in Gegenwart von Methanol und hochkonzentrierter Chlorwasserstoffsäure in einem kontinuierlichen Reaktionsverfahren vom Typ des vollständigen Mischens oder in einem Verfahren, welches sowohl kontinuierliche als auch diskontinuierliche Reaktionsstufen vom Typ des vollständigen Mischens umfaßt, entfernt wird, und der so gebildete α-L-Aspartyl-L-phenylalanin-methylester in Form des wenig löslichen Hydrochlorids abgetrennt wird, wobei das kontinuierliche Reaktionsverfahren bei einer Temperatur im Bereich von 15°C bis 40°C durchgeführt wird.

2. Verfahren nach Anspruch 1, wobei das kontinuierliche Reaktionsverfahren bei einer Temperatur im Bereich von 20 bis 35°C durchgeführt wird.

## Revendications

1. Procédé de production du chlorhydrate d'ester méthylique d'α-L-aspartyl-L-phénylalanine, lequel comprend l'élimination du groupement formyle de l'ester méthylique de N-formyl-α-L-aspartyl-L-phénylalanine en présence de méthanol et d'acide chlorhydrique de concentration élevée par un procédé de réaction continue de type mélange total ou par un procédé comprenant à la fois des étapes de réactions continue et discontinue de type mélange total, ainsi que la séparation de l'ester méthylique d'α-L-aspartyl-L-phénylalanine ainsi produit sous la forme du chlorhydrate faiblement soluble, dans lequel on réalise ledit procédé de réaction continue à une température comprise dans la gamme de 15°C à 40°C.

2. Procédé selon la revendication 1, dans lequel on réalise ledit procédé de réaction continue à une température comprise dans la gamme de 20°C à 35°C.
